# EUROPEAN PATENT APPLICATION

(11) **EP 0 545 628 A2**
(43) Date of publication of application: **09.06.1993**
(21) Application number: 92310799.9
(22) Date of filing: 25.11.1992
(51) Int. Cl.: A61N 1/365, A61N 1/37

(54) **Method and apparatus for comparing the ST segment of an electrocardiogram with a stored template**

(30) Priority: 29.11.1991 US 800464
(71) Applicant: CARDIAC PACEMAKERS, INC., St. Paul, Minnesota 55112-5798 (US)
(72) Inventor: Potocki, John C., Hanover, Pennsylvania 17331 (US)
(74) Representative: MacGregor, Gordon

(57) **Abstract**

A method of comparing the sensed ST segment of an electrocardiogram with a stored template, for differences therebetween and providing a signal when the differences reach predetermined values, characterised in that the stored template is provided by using an implanted pacemaker to sense initial electrocardiogram signal information representative of the ST segment of an electrocardiogram and storing the information in the pacemaker, or an external programmer as said template, and subsequently using the pacemaker to sense information representative of the ST segment of an electrocardiogram for comparison with said template. The invention also resides in apparatus for carrying out the method.

## Description

This invention relates to a method and apparatus for comparing the ST segment of an electrocardiogram with a stored template.

Commercially available Holter systems are typically used to compare the ST segments of intermittently sampled electrocardiogram waveforms with stored templates of acceptable waveforms. When a sampled waveform falls outside of acceptable preset amplitude or duration ranges, or when the polarity of the segment reverses, then a real-time segment of the electrocardiogram occurring at the time of the shift is downloaded for printing in report format.

In contrast, the present invention uses existing implanted pacemaker circuitry to obtain electrocardiograms and automatically to assess for shifts in ST segment changes, such as polarity, amplitude or duration.

Cardiac pacemaker systems typically are comprised of an implantable unit which contains pacemaker circuitry, at least one electrically conducting lead for disposition in proximity to cardiac tissue, and a non-implantable, programmable base unit for receiving telemetered signals from the implanted pacemaker and for sending signals to the pacemaker to alter the settings therein. A typical implanted pulse generator system is disclosed in US-A-4 388 927.

The implanted lead used within such a system typically measures an electrocardiogram via unipolar or bipolar sensing. The pacemaker is preprogrammed to assess the sensed waveform of the electrocardiogram and to determine therefrom the necessity for application of at least one stimulating pulse to the heart. A stimulating pulse is typically delivered by a stimulating electrode mounted at the distal tip of the implanted lead.

Typical sensing electrodes sense the electrocardiogram signal discharged by myocardial tissue as rhythmic waves of electrical activity are transferred within the conduction pathways of the heart. A recording of this discharge formulates the standard electrocardiogram. As different regions of the heart become activated and then repolarized, characteristic fluctuations in electrical activity are recorded. These fluctuations have been denoted the P, Q, R, S and T portions of the electrocardiogram.

Alterations in polarity, amplitude or duration of the electrocardiogram signal are caused by disruption or asynchrony in electrical discharge and repolarization within the fibrils of the myocardium and related conductive pathways. Such alterations may be caused by damage to myocardial tissue, which typically manifests itself by shifting of the ST segment of the electrocardiogram waveform.

Myocardial ischemia is damage to tissue within the heart itself due to an inadequate blood supply to a region of the heart wall. Various pathological conditions may cause ischemia to occur within a portion of the heart, the most common being a blockage of blood flow within an artery due to the presence of a clot emanating from plaque buildup, or a blockage of flow due to spasm of the arterial wall. If the blockage persists a sufficient period of time, the myocardial tissue becomes starved for oxygen and is irreparably damaged. Tissue damaged in this manner is not capable of conducting the stimulating electrical wave which causes contraction of the heart. Thus, the waveform of the electrocardiogram is altered by the presence of an ischemic region. Such damage may manifest itself as an increase in the amplitude of the T-wave, an elongation of the period between the occurrence of the S and T portions of the waveform, an inversion in polarity of the T-wave or as either an elevation or a depression of the appearance of the ST segment of the electrocardiogram when compared to a previous electrocardiogram. Typically, it is elevation or depression of the ST segment which is evident when an ischemic waveform is compared to a baseline or reference waveform.

An object of the present invention is to provide a new and improved method of and an apparatus for storing data relating to the ST segment of an electrocardiogram either externally or within an implanted pacemaker unit to be retrieved at a later date via telemetry for the purpose of assessing ST segment changes.

The present invention provides apparatus for use in comparing the sensed ST segment of an electrocardiogram with a stored template, the apparatus comprising a programmer, an implantable pacemaker of the type capable of sensing electrocardiogram signal information and telemetering such information to the programmer, a pacing lead having sensing and pacing electrodes, the pacemaker, or programmer including means for storing initially sensed electrocardiogram signal information representative of the ST segment of the electrocardiogram, the programmer having comparing means for comparing subsequently sensed information representative of the ST segment of an electrocardiogram with said initially sensed and stored information, and means for providing a signal when differences between the compared information reach stored predetermined values.

The invention also provides a method of comparing the sensed ST segment of an electrocardiogram with a stored template, for differences therebetween and providing a signal when the differences reach predetermined values, characterised in that the stored template is provided by using an implanted pacemaker to sense initial electrocardiogram signal information representative of the ST segment of an electrocardiogram and storing the information in the pacemaker, or an external programmer as said template, and subsequently using the pacemaker to sense information representative of the ST segment of an electrocardiogram for comparison with said template.

Conventional pacemaker circuitry is utilised in conjunction with standard methodology for telemetry. The patients' intrinsic cardiac electrical activity is sensed and stored to formulate a baseline electrocardiogram for subsequent comparison. This electrocardiogram may be stored within the pacemaker unit or downloaded to a standard external telemetry base unit, where it is stored for comparison to subsequent samples. At periodic intervals, subsequent electrocardiograms are recorded using the existing circuitry of the pacemaker unit. The portion of the electrocardiogram representing the ST segment is selected from these samples and compared to that portion of a previous baseline electrocardiogram.

The presence of ischemia within the myocardial walls of a patient manifests itself in a variety of clinically accepted manners. Within the ST segment, changes in polarity, duration or amplitude of the segment can indicate the presence of an ischemic region of myocardial tissue.

The aforementioned objects and advantages of the invention will become subsequently apparent and reside in the details of construction and operation as more fully hereinafter described and claimed, reference being had to the accompanying drawings forming a part thereof, wherein like numerals refer to like parts throughout.

### DESCRIPTION OF THE DRAWINGS

Figure 1 depicts a plan view of the relationship between the major components required to execute the method of the present invention;
Figures 2A to 2F depict electrocardiograms illustrating the various electrical phenomena detected by the present invention;
Figure 3 depicts a functional block diagram of the sequence of steps necessary to practice the method of the present invention.

### DESCRIPTION OF THE PREFERRED EMBODIMENT

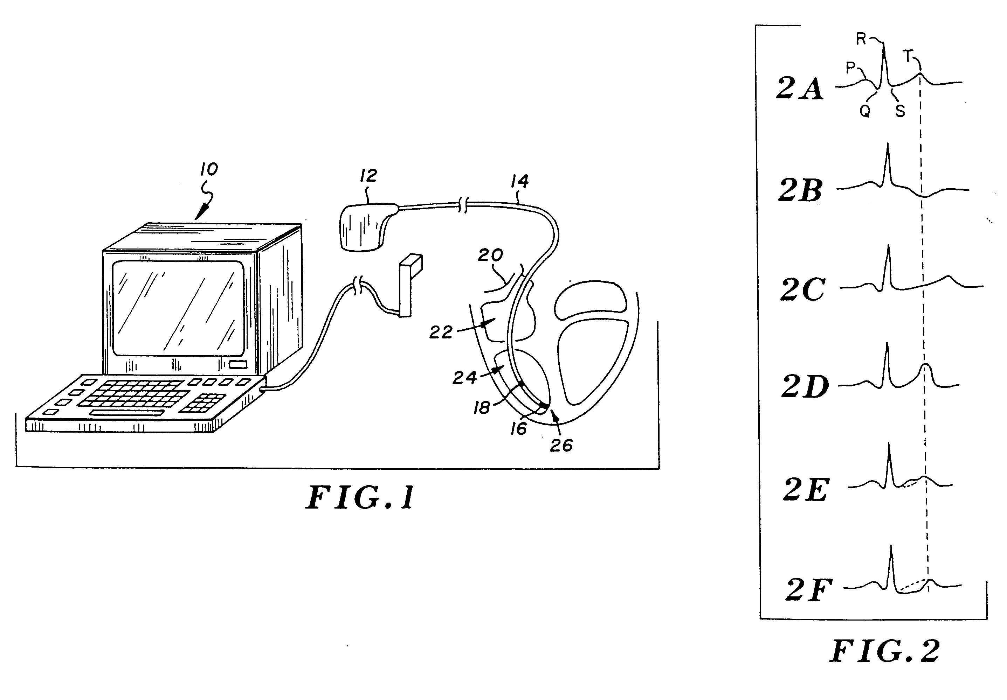

Figure 1 generally depicts the apparatus of the present invention, which may comprise a variety of existing cardiac pacing/telemetry systems. In such a system, an external programmer unit 10 is configured to receive a telemetry signal from an implanted cardiac pacemaker unit 12 affixed to a conventional cardiac pacing lead 14. Such a lead typically includes a pacing electrode 16 positioned at its distal end. At least one sensing electrode 18 is positioned along pacing lead 14 at such distance from its distal end to avoid electrical interference from pacing electrode 16. This lead is typically inserted through the superior vena cava 20 and right atrium 22 in order that the distal pacing electrode 16 be positioned within the right ventricle 24 in the region of the apex 26.

An example of an external programmer system which is useful for executing the present invention is disclosed in US-A-4 562 841. This external programmer includes a telemetry system for receiving transmitted electrocardiogram data from an implanted pacemaker then transmitting 100 kHz radio frequency (RF) pulses to a receiver within the pacemaker, which formats the transmission into proper sequence for interpretation by the pacemaker circuitry.

An alternative external monitoring station for the analysis of electrocardiogram signals is disclosed in US-A-4 173 971. In normal operating status, the electrocardiogram of a patient is recorded for a predetermined time period and the start signals are automatically downloaded to the monitoring station upon expiration of the time period. Real time electrocardiogram signals are also transmitted and a report is automatically generated containing selected portions of the electrocardiogram. The monitoring station may be programmed to return its recording apparatus to the normal operational mode for another predetermined time period, or it may continue to receive and analyse a real time electrocardiogram signal. When storing the electrocardiogram signal, the system is capable of compressing the time required to reproduce signals as compared to the time originally required to record them, thus saving memory. A communications interface permits the reproduced signals to be monitored.

An example of a programmable digital cardiac pacer which may be used in conjunction with the present invention is disclosed in US-A-4 388 927. Although this pacer includes separate digital filter circuits for sensing atrial and ventricular activity, the method of the present invention may be used with pacers that are limited either to atrial or ventricular activity sensing. The memory of the '927 pacer is capable of storing parameter data which is subsequently used by digital filters for identifying the various components of cardiac activity, such as P, R and T waves, as well as for identifying aberrant contractions. This particular system is also capable of generating a stimulating pulse when a P-wave or natural R-wave is not sensed during the sampling period and it uses T-wave parameters in the ventricular filter to verify adequate capture of tissue during administration of the stimulating pulse. The system is also capable of generating a train of stimulating signals at a higher rate than an innate rate which is generated during tachycardia, in an effort to break the tachycardic cycle. System parameters such as ventricular rate, stimulating pulse amplitude, pulse duration, atrial and ventricular filter parameters, P-R delay intervals, and others, may be externally programmed. Pacemakers, such as that disclosed in US-A-4 388 927, typically include circuitry for accessing memory for comparing the input signals from the sensing leads disposed on or within the heart to stored values for the various components of the electrocardiogram.

An intracardiac catheter/lead designed to detect electrical signals emanating from any part of the heart wall is disclosed in US-A-4 681 117. This electrode catheter is passed transvenously into the right ventricular cavity, or transarterially into the left ventricular cavity. Unipolar, bipolar, or multipolar electrode means are disposed along the catheter body in order that sensing electrode surfaces will detect electrical current from regions of the heart wall without sensing the full electrocardiogram signal. It further includes means to prevent the sensing electrodes from contacting the endocardial surface and, thus, to enable the operator to diagnose subendocardial and transmural ischemia.

Referring now to Figure 2, representative electrocardiogram (ECG) signals are shown which demonstrate the types of ST segment changes assessed by the method of the present invention. Figure 2A is a normal PQRST electrocardiogram signal as would be detected by an implanted pacemaker. Variation in normal electrical activity is represented on the ordinate and time is represented on the abscissa. The duration of the time interval between the S and T portions in this particular electrocardiogram depicts the interval that may be expected in a normal individual. The divergence of the T signal from an isoelectric value is also normal.

Figures 2B through 2F depict various alterations of the signal in the ST segment of the electrocardiogram which appear when ischemic tissue is present in the heart. These alterations include:

Figure 2B depicts an ischemic electrocardiogram signal in which the polarity of the T-wave is inverted.

Figure 2C depicts an electrocardiogram signal in which the duration of the ST segment is greatly increased beyond normal ranges.

Figure 2D depicts an electrocardiogram signal having an aberrantly elevated amplitude of the T-wave.

Figure 2E depicts ST segment elevation and Figure 2F depicts ST segment depression, either of which may be an indication of myocardial ischemia.

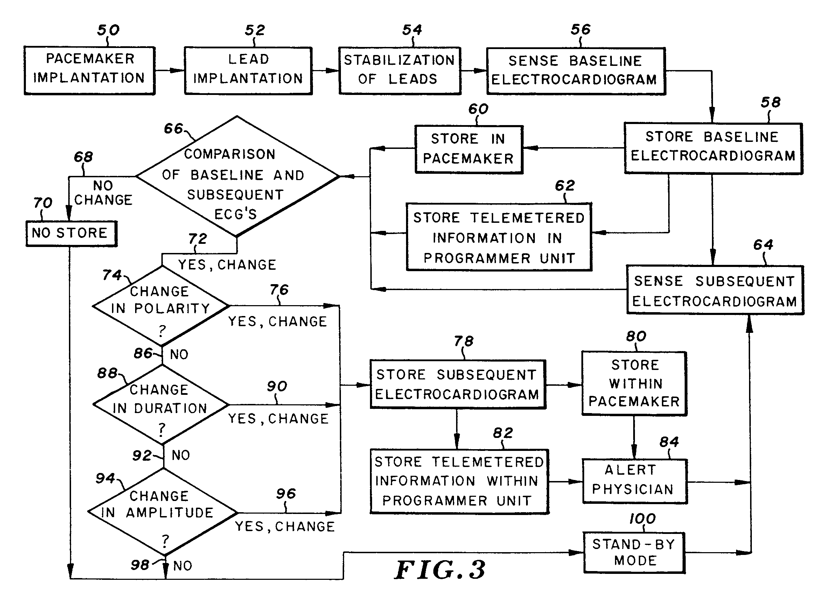

Figure 3 provides a functional block diagram denoting the steps of a method utilising the present invention. A conventional cardiac pacemaker 12 and lead 14 are implanted into a patient in the manner known in the art, as indicated at Blocks 50 and 52. The pacemaker 12 is of a type which includes telemetry capability. The lead 14 includes both sensing and pacing electrodes. A sufficient period of time is allowed at Block 54 to enable the distal tip of the pacing lead 14 to be positionally stabilised within the right ventricle. This typically occurs because of tissue encapsulation or ingrowth over a period of four to nine weeks. After such a period of stabilisation, the relative positioning of the sensing electrode is subject to minimal variation. Thus, both motion artifact and signal variation are minimised.

After the lead 14 is adequately stabilised, the pacemaker circuitry is activated by the sensing of an electrocardiogram signal, as at Block 56. This electrocardiogram signal is stored as a baseline waveform at Block 58. It may be retained within the pacemaker, as at Block 60, or it may be telemetered to an external programmer unit for storage as a reference or baseline signal, as at Block 62. Subsequent electrocardiogram signals may be recorded when deemed necessary, as indicated at Block 64.

The method involves recording the subsequent electrocardiogram signal then comparing it (Block 66) to the baseline or reference waveform taken at an earlier point in time (Block 56) and stored in either the circuitry of the cardiac pacer 12 (Block 60) or that of the external programmer 10 (Block 62). The method evaluates the ST segment of the electrocardiogram signal for any of the aberrancies depicted in Figures 2B-2F. The presence of any one of these changes in the ST segment will trigger circuitry to download this electrocardiogram sample or a portion thereof and, thus, provide a signal indication of electrocardiogram changes. Although any one of these aberrancies may signal a change, more than one may be present in the same electrocardiogram sample.

If there has been no change in the ST segment of the electrocardiogram waveform, as at Line 68, this waveform is not stored within the circuitry of the device, as indicated at Block 70. The device then returns to a "standby" mode (Block 100) in which it can receive and process a new request to sense a subsequent electrocardiogram. If, however, the sample waveform differs by a degree that exceeds the programmed normal ranges for signal variation, as at Line 72, the waveform is evaluated at Block 74 for a change in polarity. If it is determined that the polarity of the T-wave has changed, as at Line 76, the waveform is downloaded for storage at Block 78 in either the pacer (Block 80) or programmer unit (Block 82). In either event, when a subsequently sampled electrocardiogram is stored because it exceeds normal parameters, a signal indication is automatically provided, as at Block 84. With no limitation intended, one way in which this occurs is via automatically printing both the baseline and subsequent electrocardiograms on an oscilloscope-type screen or on standard electrocardiogram chart paper.

When there is no change in polarity, as at Line 86, the waveform is next evaluated for a change in duration of ST segment, as at Block 88. If it is determined that the ST segment length has elongated beyond normal ranges, as at Line 90, this waveform is stored at Block 78 in the previously described manner.

Finally, if there is no change in either polarity or duration, as at Line 92, the electrocardiogram is analysed, at Block 94, for the presence or absence of change in amplitude. If an amplitude change is present, the waveform is downloaded (at Line 96) for storage at Block 78, as previously described. If there has not been a significant change from the initial sensed electrocardiogram after performing all three assessments for change in polarity, change in duration and change in amplitude, the waveform is not stored, as at Line 98. The device then returns to a stand-by mode, indicated by Block 100, wherein it is capable of receiving and processing a new request to sense a subsequent electrocardiogram (Block 64).

In a routine office visit or under emergency conditions, a pacemaker-implanted patient can sample a fresh electrocardiogram for comparison to a baseline waveform. Using the method of the present invention, the ST segment of the two electrocardiograms are compared. If a significant shift has occurred in the waveform, both the reference (baseline) and subsequent (sample) waveforms are displayed.

## Claims

1. Apparatus for use in comparing the sensed ST segment of an electrocardiogram with a stored template, the apparatus comprising a programmer, an implantable pacemaker of the type capable of sensing electrocardiogram signal information and telemetering such information to the programmer, a pacing lead having sensing and pacing electrodes, the pacemaker, or programmer including means for storing initially sensed electrocardiogram signal information representative of the ST segment of the electrocardiogram, the programmer having comparing means for comparing subsequently sensed information representative of the ST segment of an electrocardiogram with said initially sensed and stored information, and means for providing a signal when differences between the compared information reach stored predetermined values.

2. Apparatus according to Claim 1, wherein the comparing means compares differences in the durations of the ST segments, the amplitudes of the ST segments and/or the polarities of the ST segments.

3. A method of comparing the sensed ST segment of an electrocardiogram with a stored template, for differences therebetween and providing a signal when the differences reach predetermined values, characterised in that the stored template is provided by using an implanted pacemaker to sense initial electrocardiogram signal information representative of the ST segment of an electrocardiogram and storing the information in the pacemaker, or an external programmer as said template, and subsequently using the pacemaker to sense information representative of the ST segment of an electrocardiogram for comparison with said template.

4. A method according to Claim 3, wherein the differences compared are the durations of the ST segments, the amplitudes of the ST segments and/or the polarities of the ST segments.
